# NEUE EUROPÄISCHE PATENTSCHRIFT

(11) **EP 2 726 113 B2**
(45) Veröffentlichungstag und Bekanntmachung des Hinweises auf die Entscheidung über den Einspruch: **20.07.2022**
(45) Hinweis auf die Patenterteilung: 22.05.2019
(21) Anmeldenummer: 12734803.5
(22) Anmeldetag: 29.06.2012
(51) Int. Cl.: A61L 15/20, A61L 15/42

(54) **WUNDVERSORGUNGSPRODUKT**
WOUND CARE PRODUCT
PRODUIT DE SOIN POUR PLAIES

(30) Priorität: 30.06.2011 DE 102011106046
(43) Veröffentlichungstag der Anmeldung: 07.05.2014
(73) Patentinhaber: Paul Hartmann AG, 89522 Heidenheim (DE)
(72) Erfinder: CROIZAT, Pierre, 89542 Herbrechtingen (DE); JUNGINGER, Martin, 89568 Hermaringen (DE); STELZER, Sandra, 89522 Heidenheim (DE)
(74) Vertreter: Ter Meer Steinmeister & Partner
(86) Internationale Anmeldenummer: PCT/EP2012/002743
(87) Internationale Veröffentlichungsnummer: WO 2013/000579

(56) Entgegenhaltungen:
- EP-A1- 2 338 529
- EP-A2- 0 901 795
- WO-A1-02/28447
- WO-A1-02/47737
- WO-A1-2010/000451
- DE-A1-102008 031 183
- US-A- 4 813 942
- US-A1- 2004 096 489
- US-A1- 2005 079 147
- "Medical Devices: Guidance document", , 3 December 2009 (2009-12-03), XP055023202, Retrieved from the Internet: URL:http://ec.europa.eu/health/medical-dev ices/files/meddev/2_1_3_rev_3-12_2009_en.p df [retrieved on 2012-03-28]
- Dr. Elke Lehmann ET AL: "Abgrenzungsprobleme aus der Sicht des BfArM anhand aktueller Beispiele Bundesinstitut für Arzneimittel und Medizinprodukte", , 26 March 2006 (2006-03-26), XP055312516, Retrieved from the Internet: URL:http://www.gd-online.de/german/veranst alt/images2007/E_Lehmann_11.GD_Jahrestagun g_26.03.2007.pdf [retrieved on 2016-10-20]

## Beschreibung

Die vorliegende Erfindung betrifft ein Wundversorgungsprodukt, insbesondere für die feuchte Wundbehandlung von Wunden während der Entzündungs- und der Granulationsphase der Wundheilung.

Die Heilung von Hautwunden beruht auf der Fähigkeit der Haut, Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase). Es hat sich gezeigt, dass eine Abheilung der Wunde durch eine moderne, feuchte Wundbehandlung besonders gefördert wird. Im Rahmen der feuchten Wundbehandlung werden unter anderem Wundauflagen mit einer Schaumschicht eingesetzt. Solch eine Schaumschicht bietet nachwachsendem Gewebe eine Matrix, die die Wundheilung stimuliert, und kann gleichzeitig größere Mengen Wundexsudat aufnehmen und binden. Außerdem hat es sich bewährt, für die Feuchthaltung von Wunden Hydrogele einzusetzen, die zudem Hautirritationen am Wundrand verringern.

Es ist seit Langem bekannt, dass es bei der Wundheilung, insbesondere während der Entzündungs- und der Granulationsphase der Wundheilung, zu Störungen kommen kann. Im Zusammenhang mit der vorliegenden Erfindung schließt der Begriff Wunde den Wundgrund ein. In der Wunde kann sich Wundexsudat befinden. Die Wundheilung kann dabei vom pH-Wert einer Wunde bzw. vom pH-Wert des in der Wunde enthaltenen Wundexsudats beeinflusst werden. Gesunde Haut weist üblicherweise einen pH-Wert im Sauren auf, der ungefähr zwischen pH 4,0 bis pH 5,7 liegt.

Bei Wunden, welche eine gestörte Wundheilung aufweisen, insbesondere bei chronischen Wunden, kann häufig ein alkalischer pH-Wert der Wunde oder des Wundexsudats beobachtet werden. Die Messung kann beispielsweise durch eine in-vitro-Messung des pH-Werts von Wundexsudat erfolgen. Eine Verschiebung des pH-Wertes vom Sauren ins Alkalische kann beispielsweise durch die Vermehrung von Bakterien oder durch die Bildung von Nekrosen verursacht werden.

Nekrosen und pathologische Mikroorganismen können auf den physiologischen Metabolismus während des Wundheilungsgeschehens einwirken. Dies führt häufig zu lokaler Hypoxie, und dann zum weiteren Abbau umliegenden Gewebes. Das entstehende alkalische Milieu kann weitere gewebeabbauende Prozesse begünstigen. Weiterhin kann das alkalische Milieu die Vermehrung weiterer pathogener Mikroorganismen stimulieren und so die Wundheilung zusätzlich behindern. Als chronische Wunden werden im Rahmen dieser Erfindung Wunden bezeichnet, die nicht in einem erwarteten Zeitraum von 4 bis 6 Wochen verheilen.

Im Stand der Technik sind zahlreiche den pH-Wert einer Wunde verändernde Produkte bekannt. In der US4813942 ist eine Wundauflage beschrieben, deren Klebeschicht Polyisobutylen und ein Hydrocolloid aufweist und den pH-Wert der Wunde auf pH 4,8 bis pH 6,5 einstellen kann. Die EP1322348 beschreibt gleichfalls ein den pH-Wert einer Wunde erniedrigendes Klebemittel, das unter anderem mit Zitronensäure und anorganischen Puffern versehen sein kann. Die EP0506300 beschreibt einen Wundverband, der ein hydrophiles Gel umfasst, wobei das Gel Polyurethan und Poly-(N-vinyllactam) mit einem K-Wert größer 60 und mehr als 1,4 Moläquivalenten saure Gruppen aufweist. In der WO01/39582 wird ein den pH-Wert von Wunden beeinflussendes Puffersystem im pH-Bereich 2 bis 5,5 beschrieben, das insbesondere gegen den Hefepilz Candida albicans wirksam ist. Das Pufferssystem wird dabei in einem die Haut abdeckenden Artikel wie einer Wundauflage, einer Windel oder einem schleimhautabdeckenden Artikel verwendet und umfasst bevorzugt einen Superabsorber mit den pH-Wert stabilisierenden Eigenschaften. Die EP1341561 beschreibt ein mehrschichtiges Wundverbandsmaterial, das eine Hydrogel- und eine Barriereschicht umfasst, wobei die Barriereschicht ein Material umfasst, dessen Löslichkeit vom pH-Wert abhängt. Das Material ist bei pH 4 unlöslich, bei pH 8 jedoch wasserlöslich. Somit wird bei einem alkalischen pH-Wert die Barriereschicht durchlässig, um das anfallende Wundsekret in einer weiteren Schicht aufzunehmen. Das wundseitige Hydrogel enthält ein schwaches, wasserlösliches Puffersystem.

Die Anmeldung EP901795 offenbart feste bioabsorbierbare Materialien aus einer Kollagenmatrix die Puffersubstanzen enthalten und als Wundauflagen verwendet werden können. Diese Kollagen-Wundauflagen werden durch Kontakt mit Wundexsudat aufgelöst und vom Körper resorbiert. Sie verbleiben bis zur vollständigen Resorption auf der Wunde und sind daher zur Entfernung von zerstörtem Gewebe oder Wundflüssigkeit nicht geeignet.

Die Anmeldung EP2338529 offenbart mehrschichtige Wundauflagen mit einer Hydrogelmatrix als Wundkontaktschicht und einer zusätzlichen absorbierenden Schicht, die einen Schaum umfassen kann. Diese Anmeldung offenbart keine Puffersubstanzen in der Wundauflage.

Weiterhin werden beispielsweise mit den europäischen Patenten EP457977, EP486522, EP541390, EP541391, EP570430, EP665856, EP691113, EP693913 oder EP1082146 Wundauflagen mit verschiedenen Konstruktionen beschrieben, die als absorbierende Schicht einen Polyurethanschaum umfassen.

Darüber hinaus sind mit den europäischen Patenten EP855921 und EP1156838 Wundauflagen bekannt, die einen Polyurethanschaum umfassen, der mit einem hydrophoben Silikongel beschichtet ist. Dieses Silikongel soll die Verklebung der Wunde mit dem Polyurethanschaum verhindern.

Weiterhin werden mit den internationalen Anmeldungen WO02/38 097, WO02/47761, WO03/011352, WO 03/086255, WO2004/052415 oder EP1658865 Wundauflagen beschrieben, die ein Hydrogel und einen Polymerschaum umfassen.

In der Patentanmeldung des Anmelders DE102008031183 ist gleichfalls eine mehrschichtige bzw. mehrlagige Wundauflage mit einer Wundkontaktschicht als erster Schicht und mindestens einer zweiten Schicht als absorbierende Schicht, die einen hydrophilen Polyurethanschaum umfasst, beschrieben. Die Wundkontaktschicht kann ein Hydrogel auf Basis eines Polyharnstoff/Polyurethan-Copolymers sein.

GB-A-2082911 beschreibt pharmazeutische Salben zur Anwendung auf Wunden. Diese Salben enthalten eine Mischung aus Aminosäuren und Apfelsäure und weisen einen pH von 6,5 bis 8 auf.

EP564307 beschreibt Windeln, Hygienetücher, Zusammensetzungen und Cremes, die Puffersubstanzen zur Verringerung von Hautreizungen enthalten, wie sie bei Hautkontakt mit Körperflüssigkeiten auftreten können. Die Zusammensetzungen stabilisiern den pH-Wert im Bereich von 4,5 bis etwa 6,0. Dieses Dokument enthält keine Offenbarung zu Wundversorgungsprodukten enthaltend Puffersubstanzen.

Der vorliegenden Erfindung liegt die Aufgabe zugrunde, ein verbessertes Wundversorgungsprodukt zur Verfügung zu stellen, insbesondere soll die Wundheilung chronischer Wunden verbessert und die Nachteile des Standes der Technik überwunden werden. Ausgehend von dem Markt zur Verfügung stehenden Wundauflagen ist es eine weitere Aufgabe der vorliegenden Erfindung, eine verbesserte Wundauflage bereitzustellen, die eine hohe Absorptionsfähigkeit für Wundexsudat aufweist und insbesondere zur Wundheilung in der exsudativen Phase oder der Granulationsphase eingesetzt werden kann. Darüber hinaus soll eine Wundauflage bereitgestellt werden, die den pathologischen Zustand einer Wunde derart beeinflusst, dass ein schneller und mit geringer Narbenbildung verbundener Wundheilungsverlauf stattfinden kann. Hierfür soll die Wundauflage insbesondere ein gutes Absorptionsvermögen aufweisen.

Zur Lösung dieser Aufgabe schlägt die Erfindung ein Wundversorgungsprodukt gemäß Anspruch 1 und ein Verfahren zur Herstellung eines Wundversorgungsprodukts gemäß Anspruch 8 vor. Das erfindungsgemäße Wundversorgungsprodukt umfasst Puffersubstanzen und eine Wundauflage, wobei die Wundauflage einen Schaum aufweist und der pH-Wert der sich bei Lösung der Puffersubstanzen in demineralisiertem Wasser bei 37°C ergebenden Pufferlösung zwischen pH 3 und pH 7 liegt, dadurch gekennzeichnet, dass das Wundversorgungsprodukt eine Wundkontaktschicht aufweist, welche ein Hydrogel umfasst, wobei die Puffersubstanzen in dem Schaum und in dem Hydrogel vorgesehen sind, wobei die Puffersubstanzen Zitronensäure und Zitrat als Puffersubstanzen umfassen und wobei der Schaum ein hydrophiler Polyurethanschaum ist. Die Wundauflage ist mehrschichtig aufgebaut.

Der Begriff Pufferlösung bezeichnet im Rahmen der Erfindung eine Zusammensetzung, wobei die Zusammensetzung eine schwache Säure und ihre korrespondierende Base oder wobei die Zusammensetzung eine schwache Base und ihre korrespondierende Säure umfasst. Die Gegenionen der korrespondierenden Base können zum Beispiel Natriumionen sein. Die Pufferlösung kann insbesondere aus einer schwachen Säure und dem Salz dieser Säure bestehen. Die Zusammensetzung liegt in einer wässrigen Lösung vor. Die einzelnen Substanzen der jeweiligen Zusammensetzung, also Säure und Base oder Salz, werden dabei als Puffersubstanzen bezeichnet. Die Puffersubstanzen sind bevorzugt nicht-toxische, hautverträgliche und physiologisch unbedenkliche Stoffe. Der pH-Wert der Pufferlösung ergibt sich aus dem Protolyse-Gleichgewicht des Pufferpaares aus Säure und Base oder Salz. Der pH-Wert einer Pufferlösung kann in einer guten Näherung gemäß der Henderson-Hasselbalch-Gleichung berechnet werden. Die vorgenannte Gleichung besagt, dass der pH-Wert einer Pufferlösung gleich dem pKs-Wert der Säurekomponente der Pufferlösung plus dem dekadischen Logarithmus des Quotienten der Konzentration der korrespondierenden Base zur Säurekonzentration ist. Aus der Definition der Henderson-Hasselbalch-Gleichung ergibt sich folglich, dass bei gleichen Konzentrationen der Puffersubstanzen der pH-Wert gleich dem pKs-Wert ist. Im Rahmen der vorliegenden Erfindung ist die Verwendung von gleichen Konzentrationen der jeweiligen Puffersubstanzen bevorzugt. Solchenfalls entspricht der pH-Wert der sich bei Lösung der Puffersubstanzen ergebenden Pufferlösung in guter Näherung dem pKs-Wert der Säurekomponente der Puffersubstanzen. Somit kann die Henderson-Hasselbalch-Gleichung genutzt werden, um mittels der pKs-Werte von Puffersubstanzen den pH-Wert der sich ergebenden Pufferlösung abzuschätzen.

Bei einem erfindungsgemäßen Wundversorgungsprodukt wird der pH-Wert der sich bei Lösung der Puffersubstanzen ergebenden Pufferlösung jedoch nicht berechnet, sondern mittels einer pH-Wert Messung bestimmt. Für eine solche Messung werden die Puffersubstanzen mit einer Konzentration von 0,1 M in einem Liter demineralisiertem Wasser mit einer Temperatur von 37 °C unter Rühren vollständig gelöst, wodurch sich eine Pufferlösung ergibt. Das Rühren der Lösung kann zum Beispiel mit einem handelsüblichen Magnetrührer durchgeführt werden. Der pH-Wert der Pufferlösung kann in an sich üblicher Weise mit einem auf Potentiometrie beruhendem handelsüblichen pH-Meter gemessen werden. Das pH-Meter sollte vor der Messung mittels handelsüblicher Eichlösungen geeicht werden und entsprechend der Bedienungsanleitung des Herstellers verwendet werden. Die Messung des pH-Werts der Pufferlösung erfolgt durch Eintauchen der Messelektrode des pH-Meters in die Pufferlösung, wobei die Pufferlösung gerührt wird. Anschließend erfolgt ein Ablesen des gemessenen pH-Werts auf der Anzeige des pH-Meters.

Eine solche Pufferlösung ist dazu geeignet, den pH-Wert einer Flüssigkeit in einem bestimmten pH-Bereich zu stabilisieren, wobei im Rahmen der vorliegenden Erfindung dieser pH-Bereich als Pufferbereich bezeichnet wird. Die vorgenannte Flüssigkeit kann Wundexsudat sein.

Der Pufferbereich einer Pufferlösung ist in an sich üblicher Weise definiert als ein pH-Bereich mit einer unteren und einer oberen Grenze. Die untere Grenze des Pufferbereichs ist im vorgenannten Fall gleicher Konzentrationen der Puffersubstanzen der pKs-Wert der sich bei einer Lösung der Puffersubstanzen ergebenden Pufferlösung minus eine pH-Einheit. Die obere Grenze des Pufferbereichs ist im vorgenannten Fall gleicher Konzentrationen der Puffersubstanzen der pKs-Wert der vorgenannten Pufferlösung plus eine pH-Einheit.

Weiterhin haben Pufferlösungen die Eigenschaft, den pH-Wert bei Zugabe einer Flüssigkeit mit einem anderen pH-Wert als der pH-Wert der Pufferlösung, über längere Zeit im Pufferbereich zu stabilisieren. Ein quantitatives Maß für die vorgenannte pH-Wert stabilisierende Eigenschaft ist die Pufferkapazität der Pufferlösung.

Die Pufferkapazität ist im Rahmen der vorliegenden Erfindung durch die Menge in mol an NaOH definiert, die in Titrationsexperimenten nötig ist, um den pH-Wert einer Flüssigkeitsmenge von 15 ml demineralisiertem Wasser pro g Produkt, welches Puffersubstanzen enthält, um eine pH-Stufe zu verändern. Die Menge an NaOH wird über den Verbrauch an 0,1 M NaOH bestimmt und in mol angegeben.

Gemäß einer Ausführungsform der Erfindung umfasst das Wundversorgungsprodukt Puffersubstanzen, wobei der pH-Wert der sich bei Lösung der Puffersubstanzen in demineralisiertem Wasser bei 37°C ergebenden Pufferlösung zwischen pH 3,5 und pH 6 liegt. Dies ist von Vorteil, da vorgenannte pH-Werte in dem physiologischen pH-Wert Bereich gut abheilender Wunden liegen.

Gemäß einer weiteren Ausführungsform der Erfindung umfasst das Wundversorgungsprodukt Puffersubstanzen, wobei der pH-Wert der sich bei Lösung der Puffersubstanzen in demineralisiertem Wasser bei 37°C ergebenden Pufferlösung zwischen pH 3,8 und pH 5 liegt. Dies ist von Vorteil, weil einige leicht und kostengünstig herzustellende Puffersubstanzen wie z.B. Benzoesäure/Benzoat (pKs 4,2) oder Milchsäure/Lactat (pKs 3,9) pKs-Werte in dem vorgenannten pH-Bereich aufweisen.

Die Schaumkomponente der Wundauflage weist die Puffersubstanzen auf, wobei die Puffersubstanzen insbesondere in der Polymermatrix des Schaums vorliegen. Die Puffersubstanzen werden erst durch die Absorption von Wundexsudat gelöst. Die Pufferlösung kann dann in die Wunde zurück diffundieren und dort den pH-Wert im sauren Bereich stabilisieren. Da außerdem der pH-Wert der in den Schaum absorbierten Flüssigkeit im sauren pH-Bereich stabilisiert wird, kann die Vermehrung von Keimen, die ein alkalisches Milieu bevorzugen, vermindert werden. Somit wird eine Rückkontamination der Wunde mit Keimen verhindert und die Intervalle für Verbandswechsel können bei Bedarf verlängert werden.

In einer Weiterbildung des erfindungsgemäßen Wundversorgungsprodukts sind die Puffersubstanzen in dem Schaum homogen verteilt.

Gemäß einer Ausführungsform des erfindungsgemäßen Wundversorgungsprodukts ist es vorteilhaft, wenn die Pufferkapazität des Wundversorgungsprodukts bei einer Konzentration der Puffersubstanzen von 0,05 M mindestens 0,25 mol NaOH, insbesondere mindestens 0,55 mol NaOH beträgt.

Erfindungsgemäß weist das Wundversorgungsprodukt eine Wundkontaktschicht auf, welche ein Hydrogel umfasst.

Erfindungsgemäß umfasst ein Hydrogel, das als Wundkontaktschicht dient, Puffersubstanzen. Die im Gel enthaltenden Puffersubstanzen können im Wundexsudat gelöst werden. Die so entstandene Pufferlösung kann in die Wunde diffundieren und dort den pH-Wert im sauren Bereich stabilisieren. Da außerdem die in den Schaum absorbierte Flüssigkeit im sauren pH-Bereich gepuffert wird, wird auch bei dieser Variante die Vermehrung von Keimen, die ein alkalisches Milieu bevorzugen, vermindert. Somit wird eine Rückkontamination der Wunde mit Keimen verhindert und die Intervalle für Verbandswechsel können bei Bedarf verlängert werden. Das Hydrogel kann darüber hinaus Hautirritationen verringern. Des Weiteren versorgt das Gel insbesondere trockene Wunden mit ausreichend Feuchtigkeit. Dies ist bei Verwendung mit einer trockenen Schaumwundauflage vorteilhaft. Das Hydrogel, welches Puffersubstanzen enthält, wird mit der Schaumwundauflage, welche Puffersubstanzen enthält, kombiniert. Diese Kombination gewährleistet eine besonders hohe Pufferkapazität und eine konstante Anfeuchtung der Wunde.

In einer weiteren bevorzugten Ausführungsform umfasst das erfindungsgemäße Wundversorgungsprodukt Puffersubstanzen, wobei die Pufferkapazität des Wundversorgungsprodukts bei einer Konzentration der Puffersubstanzen von 0,05 M mindestens 0,08 mol NaOH, insbesondere mindestens 0,1 mol NaOH beträgt.

Erfindungsgemäß umfasst das Wundversorgungsprodukt nach Anspruch 1 die Puffersubstanzen Zitronensäure und Zitrat.

Gemäß einer bevorzugten Ausführungsform der Erfindung erreicht die Pufferkapazität des Wundversorgungsprodukts nach spätestens drei Stunden 90% der nach 24 Stunden messbaren maximalen Pufferkapazität. Die vorgenannte schnelle Lösung der Puffersubstanzen ist besonders vorteilhaft, weil das Wundversorgungsprodukt bei einer Anwendung auf einer Wunde schnell eine hohe Pufferkapazität bereitstellen kann. Die schnelle Bereitstellung einer hohen Pufferkapazität ermöglicht es, den pH-Wert von in der Wunde befindlichem Wundexsudat von Beginn der Behandlung an zuverlässig im sauren pH-Bereich zu stabilisieren.

Weiterhin betrifft die Erfindung Verfahren zur Herstellung eines Wundversorgungsprodukts. Das Verfahren zur Herstellung eines Wundversorgungsprodukts umfasst das zur Verfügung stellen eines Schaums, das zur Verfügung stellen einer Pufferlösung mit Puffersubstanzen, wobei der pH-Wert der Pufferlösung bei 37°C zwischen pH 3 und pH 7 liegt. Weiterhin umfasst das Verfahren das zur Verfügung stellen weiterer Schichten. Anschließend wird der Schaum mit der Pufferlösung imprägniert und anschließend entweder vollständig oder teilweise getrocknet. Zur Imprägnierung wird der Schaum für mindestens 60 Sekunden in eine Puffersubstanzen enthaltende Pufferlösung (15ml/g Schaum), die Puffersubstanzen mit einer Gesamtkonzentration von 0,01 bis 0,6 M enthält, getaucht. Im Rahmen der vorliegenden Erfindung bezeichnet der Begriff Konzentration dabei jeweils die Gesamtkonzentration aller eingesetzten Puffersubstanzen. Danach wird der Schaum abgetropft und anschließend für drei Tage bei Normklima (23°Celsius, 50 % rel. Luftfeuchtigkeit, siehe DIN EN ISO 139) vollständig getrocknet. Für eine teilweise Trocknung des Schaums kann eine kürzere Trocknungszeit gewählt werden. Nach dem Imprägnieren des Schaums mit der Pufferlösung umfasst das Verfahren weiterhin das Zusammenfügen des imprägnierten Schaums und der weiteren Schichten zu einer mehrschichtigen Wundauflage. Dieses Verfahren ist besonders dafür geeignet, einem Schaummaterial nach dessen Herstellung pH-Wert verändernde Eigenschaften zu verleihen.

Es hat sich gezeigt, dass im Falle der Verwendung der Pufferlösungen Zitronensäure/ Citratpuffer, Benzoesäure/ Benzoat und Milchsäure/ Lactat für das erfindungsgemäße Herstellungsverfahren eine Konzentration von 0,03 M bis 0,6 M Puffersubstanzen besonders vorteilhaft für eine verbesserte Wundheilung ist. Für die Imprägnierung des Schaums werden vorzugsweise 15 ml der Pufferlösung pro g Schaum verwendet. Diese Menge an Flüssigkeit entspricht der Aufnahmekapazität eines hydrophilen Polyurethanschaums mit einer Dichte von 70 bis 110 kg/m3, so dass eine fast vollständige Aufnahme der Pufferlösung durch den Schaum gewährleistet werden kann. Schäume, deren Dichte von dem beschriebenen Bereich abweicht, könnten entsprechend ihrer Aufnahmekapazität imprägniert werden.

Gemäß einer weiteren vorteilhaften Ausführungsform betrifft die Erfindung ein Verfahren gemäß des obigen Verfahrens zur Herstellung eines Wundversorgungsprodukts, bei dem die Puffersubstanzen auch in einem Hydrogel enthalten sind. Das Verfahren zur Herstellung eines Wundversorgungsprodukts umfasst das zur Verfügung stellen eines Hydrogels, das zur Verfügung stellen einer Wundauflage, die einen Schaum aufweist, und das zur Verfügung stellen einer Pufferlösung mit Puffersubstanzen, wobei der pH-Wert der Pufferlösung bei 37°C zwischen pH 3 und pH 7 liegt. Weiterhin umfasst das Verfahren das zur Verfügung stellen weiterer Schichten. Die Puffersubstanzen werden in demineralisiertem Wasser gelöst und mit dem Gel vermischt, so dass sich eine Konzentration von 0,01 bis 0,6 M Puffersubstanzen für die Mischung ergibt. Weiterhin umfasst das Verfahren das Zusammenfügen der Wundauflage und des Hydrogels, wobei das Gel die Wundkontaktschicht des Wundversorgungsprodukts bildet.

Die Erfindung umfasst weiterhin das erfindungsgemäße Wundversorgungsprodukt zur Verwendung in der Behandlung von Wunden, insbesondere chronischen Wunden. Insbesondere können Wunden, die bei traumatischen Verletzungen mit Gewebeverlust auftreten, behandelt werden. Weiterhin können Geschwürwunden wie zum Beispiel Dekubitus-Wunden und Wunden, die durch venöse Insuffizienz entstehen, mit dem erfindungsgemäßen Wundversorgungsprodukt behandelt werden. Außerdem ist das erfindungsgemäße Wundversorgungsprodukt geeignet, zur Behandlung von thermischen und chemischen Wunden eingesetzt zu werden.

Es hat sich gezeigt, dass die Wundheilung bei Anwendung des erfindungsgemäßen Wundversorgungsproduktes in besonders vorteilhafter Weise beeinflusst werden kann. Die vorteilhafte Wirkung bei der Verwendung des erfindungsgemäßen Wundversorgungsprodukts bei der Therapie von Wunden tritt dabei insbesondere bei der Behandlung chronischer Wunden auf. Bei der Verwendung des erfindungsgemäßen Wundversorgungsproduktes zur Therapie von Wunden kann das Auftreten von Wundheilungsstörungen reduziert und eine Narbenbildung verringert werden. Hierbei scheinen die an sich bekannten, die Wundheilung fördernden Eigenschaften von Schaum-Wundauflagen, mit den pH-Wert stabilisierenden Eigenschaften der Puffersubstanzen in einer sich gegenseitig verstärkenden Weise zusammenzuwirken.

Generell ist eine lange Verweildauer einer mit dem Wundgrund direkt in Kontakt tretenden Komponente des Wundverbandes auf der Wunde erwünscht, da jeder Verbandwechsel den Wundheilungsprozesses stören kann. Weiterhin ist der Verbandwechsel unangenehm fürden Patienten und erhöht den Aufwand für das behandelnde medizinische Personal. Wundauflagen mit Schäumen sind für lange Verweildauern auf der Wunde geeignet, denn sie verfügen über ein hohes Absorptionsvermögen für Wundexsudat.

Verbleibt eine aus dem Stand der Technik bekannte Wundauflage, welche einen Schaum als Wundkontaktschicht aufweist, einige Tage auf der Wunde, so kann jedoch häufig eine Verschiebung des pH-Wertes von Wunde und Wundexsudat in den alkalischen pH-Bereich beobachtet werden.

Es hat sich gezeigt, dass das erfindungsgemäße Wundversorgungsprodukt im Vergleich zu herkömmlichen Wundauflagen mit Schaum mindestens genauso lange, beispielsweise mehr als zwei Tage, auf der Wunde verbleiben kann. Hierdurch werden zusätzliche Verbandswechsel vermieden, die ansonsten die Wundheilung beeinträchtigen könnten. Es konnte beobachtet werden, dass der Puffereffekt über die Verweildauer des Wundversorgungsproduktes auf der Wunde weitgehend erhalten bleibt.

Weiterhin wurde beobachtet, dass das erfindungsgemäße Wundversorgungsprodukt ein besonders wirksames Micro-Debridement ermöglicht. Unter Micro-Debridement wird das schonende Entfernen von Wundexsudat und zerstörtem Gewebe aus der Wunde beim Verbandwechsel bezeichnet. "Micro-Debridement" unterstützt das zügige Abheilen der Wunde.

Die Anwendung des erfindungsgemäßen Wundversorgungsprodukts ist besonders bei stark exsudierenden Wunden in der Entzündungs- oder der Granulationsphase der Wundheilung, insbesondere bei sekundär heilenden Wunden, indiziert. Es hat sich gezeigt, dass die pH-Wert stabilisierenden Eigenschaften des Wundversorgungsprodukts die Ablösung von Nekrosen erleichtern und die Ausbreitung von Keimen in der Wunde verringern können, was den die Wundheilung fördernden Effekt einer Schaumwundauflage in einer sich gegenseitig verstärkenden Weise verbessert. Für die Behandlung von Wunden mit dem vorliegenden Wundversorgungsprodukt empfiehlt sich in Abhängigkeit von der Stärke der Exsudation der Wunde ein Intervall von etwa 1 bis 3 Tagen für einen Verbandswechsel. Es hat sich nämlich gezeigt, dass das Micro-Debridement bei dem vorgenannten Intervall besonders schonend für die Wunde geschehen kann, ohne dass bereits starke Verwachsungen mit der Schaumschicht zu erwarten wären, die die Wunde von neuem traumatisieren könnten.

Das erfindungsgemäße Wundversorgungsprodukt umfasst einen Schaum, der ein hydrophiler Polyurethanschaum ist. Die Verwendung eines hydrophilen Polyurethanschaums ist für eine schnelle Wundheilung vorteilhaft, weil solche Schäume eine hohe Absorptionskapazität aufweisen und daher vorzugsweise in der Reinigungsphase der Wundheilung bei starker Exsudation eingesetzt werden. Ein weiterer Vorteil von Polyurethanschäumen besteht darin, dass nur geringe Scherkräfte auf eine zu behandelnde Wunde ausgeübt werden und die Wunde somit gut abgepolstert wird.

Im Zusammenhang mit der vorliegenden Erfindung wird unter einem hydrophilen Polyurethanschaum ein Polyurethanschaum verstanden, der eine Flüssigkeit in seine Polyurethanmatrix und in seine Poren aufnehmen und speichern, somit absorbieren kann, und zumindest einen Teil der aufgenommenen Flüssigkeit wieder abgeben kann. Als hydrophile Polymerschäume sind hierbei insbesondere offenporige, hydrophile Polyurethanschäume geeignet. Demgemäß umfasst eine besonders bevorzugte Wundauflage eine Schicht, die einen offenporigen, hydrophilen Polyurethanschaum umfasst. Erfindungsgemäß sollen vorzugsweise Polyurethanschäume eingesetzt werden, die eine hohe Absorptionskapazität für Flüssigkeiten von mehr als 2,5 g, bevorzugt mehr als 10 g, noch mehr bevorzugt mehr als 16 g isotonischer Salzlösung pro Gramm Schaumpolymer aufweisen. Die Absorptionsfähigkeit wird nach DIN EN 13726-1:2002 (3 min Messung) bestimmt. Ein derartiger Schaum kann Keime und Zelltrümmer absorbieren und sicher einschließen, dabei aber trotzdem weich, geschmeidig und mit einer guten Polsterwirkung auf der Wunde aufliegen.

Vorzugsweise weist der hydrophile Polyurethanschaum eine durchschnittliche Porengröße von weniger als 1000 µm, insbesondere 100 bis 1000 µm, bevorzugt 100 bis 500 µm und ganz besonders bevorzugt 100 bis 300 µm auf. Die bevorzugte Methode zur Bestimmung der Porengröße ist die Messung des Durchmessers einer Vielzahl von Poren auf einer Schnittebene, die parallel zur Wundkontaktseite der Schaumschicht bzw. des Wundversorgungsprodukts orientiert ist. Die Messung der Porengröße kann durch Betrachten der Poren in einem Licht- oder Elektronenmikroskop und Vergleich des Porendurchmessers mit einem geeigneten Maßstab erfolgen. Der Schaum kann eine homogene Porengröße oder einen Gradienten der Porengröße über die Dicke der Schaumschicht aufweisen. Bei Verwendung eines Schaums, welcher einen Gradienten der Porengröße ausweist, wird durch eine von der Wundkontaktschicht ausgehende Verringerung der Porengröße von größeren Poren an der Wundkontaktseite (mittlere Porengröße z.B. 200-300 µm) bis hin zu kleineren Poren an der im Gebrauch von der Wunde abgewandten Seite des Schaums (mittlere Porengröße z.B. 100-200 µm) eine effiziente Ableitung von Wundexsudat gewährleistet. Eine effiziente Ableitung von Wundexsudat ergibt sich, weil ein Kapillareffekt für eine besonders gute Absorption von Flüssigkeiten erzeugt werden kann. Gleichzeitig kann der Schaum eine ausreichende Menge an Feuchtigkeit für eine Wunde bereitstellen. Ein Schaum mit einem Gradienten der Porengröße über die Dicke des Schaums und einer Porengröße von weniger als 1000 µm kommt beispielsweise bei dem Produkt Permafoam der Paul Hartmann AG zum Einsatz. Darüber hinaus ist es besonders vorteilhaft, wenn die Wundauflage außerdem eine wasserdampf-durchlässige Polyurethan-Deckschicht aufweist. Weiterhin ist es vorteilhaft, wenn die wasserdampfdurchlässige Polyurethan-Deckschicht eine Wasserdampfdurchlässigkeit ("upright", gemessen nach DIN EN 13726-2 bei einer Temperatur von 37°C) von mehr als 600 g/m² in 24 h aufweist.

Denkbar und vorteilhaft ist es darüber hinaus, wenn die Wundauflage wundseitig ein netzförmiges Hydrogel aufweist. Weiterhin ist es vorteilhaft, wenn die Wundauflage wundabseitig eine Polyurethan-Deckschicht umfasst.

Des Weiteren ist es von Vorteil, wenn der Schaum eine Dichte von 70 bis 110 kg/m³ aufweist. Gemäß einer weiteren Ausführung der Erfindung kann ein hydrophober PU-Schaum mit einer Dichte von 10 bis 50 kg/m³ verwendet werden. Derartige Schäume werden insbesondere bei Wundauflagen, welche für eine Unterdruck-Therapie von Wunden vorgesehen sind, eingesetzt. In einer weiteren Ausführung der Erfindung wäre es weiterhin denkbar und vorteilhaft, Silikonschäume mit einer Dichte von bis zu 300 kg/m³ einzusetzen.

Polyurethanschaumstoffe sind üblicherweise erhältlich durch Umsetzung einer härtbaren Mischung, umfassend die Komponenten Polyisocyanat und gegenüber Isocyanat reaktiver Verbindungen, insbesondere Polyol, sowie Katalysatoren, Treibmitteln und gegebenenfalls Zusatzstoffen. Als Isocyanate können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat, hier insbesondere 4,4'-Diphenylmethandiisocyanat, Mischungen aus monomeren Diphenylmethandiisocyanaten und höherkernigen Homologen des Diphenylmethandiisocyanats, Tetramethylendiisocyanat, Hexamethylendiisocyanat, Toluylendiisocyanat oder Mischungen daraus. Als gegenüber Isocyanaten reaktive Verbindungen werden üblicherweise Polyole wie Polyetherole und/oder Polyesterole verwendet.

Als besonders vorteilhafte Ausführungen haben sich weiterhin Schaumwundauflagen gezeigt, die einen Polyurethanschaum umfassen, dessen Schichtdicke 0,1 cm bis 1,8 cm, bevorzugt von 0,3 cm bis 1,5 cm und ganz besonders bevorzugt von 0,5 cm bis 1,0 cm, aufweist. Die Schichtdicke kann an jeder Stelle der Wundkontaktschicht gleich sein oder in verschiedenen Bereichen der Wundkontaktschicht verschiedene Werte annehmen. Insbesondere ist auch vorgesehen, dass die absorbierende Schicht oder der Polyurethanschaum abgeflachte Ränder aufweist.

Bevorzugt weist das Wundversorgungsprodukt eine im Wesentlichen quadratische Grundform auf. Besonders bevorzugt ist dabei ein Größenbereich von 8 cm x 8 cm bis zu 20 cm x 20 cm. Die Dicke des Wundversorgungsprodukts beträgt bevorzugt weniger als 2 cm, wobei die Schaumschicht bevorzugt eine Dicke zwischen 0,1 cm und 1,8 cm aufweist.

Als Wundkontaktschicht findet gemäß der vorliegenden Erfindung ein zusätzliches Material Verwendung. Hierbei steht eine Wundkontaktschicht bei Verwendung der erfindungsgemäßen Wundauflage in direktem Kontakt mit der Wunde. Die Wundkontaktschicht kann einzig und allein dazu dienen, den Schaum von der zu behandelnden Wunde zu beabstanden. Die zusätzliche Schicht hat den Vorteil, bei einem Verbandswechsel eine besonders gewebeschonende Ablösung des Wundversorgungsprodukts zu gewährleisten. Die Wundkontaktschicht kann weitere Funktionen in Bezug auf die zu behandelnde Wunde ausüben. Beispielsweise kann die Wundkontaktschicht die Wunde mit Feuchtigkeit versorgen, wundrandpflegende Eigenschaften aufweisen, Hautirritationen vermindern oder antiadherent wirken.

Eine erfindungsgemäße Wundauflage weist eine Wundkontaktschicht, wobei die Wundkontaktschicht ein Hydrogel umfasst. Der Begriff Hydrogel bzw. Gel bezeichnet dabei im Rahmen der Erfindung ein feindisperses System aus mindestens einer festen und einer flüssigen Phase. Diese feste Phase bildet dabei ein schwammartiges, dreidimensionales Netzwerk, dessen Poren durch eine Flüssigkeit (Lyogel) bzw. auch ein Gas (Xerogel) ausgefüllt sind. Beide Phasen durchdringen sich dabei vollständig.

Ein derartiges Hydrogel ist dabei vorzugsweise ein amorphes Hydrogel, welches insbesondere die Komponenten Natriumchlorid, Kaliumchlorid, Claciumchlorid Dihydrat, Carbopol, Glycerin, Natrosol und Blanose aufweist. Die Komponenten des Gels sind mit einer wässrigen Pufferlösung, welche den pH-Wert im sauren Bereich puffern kann, vermischt. Vorzugsweise umfasst diese wässrige Pufferlösung dabei äquimolare Konzentrationen der Puffersubstanzen, also das gleiche Stoffmengenverhältnis von Säure und Base.

Eine weitere vorteilhafte Rezeptur für ein Hydrogel umfasst 20-60 Gewichtsprozent Propylenglykol, 3-10 Gewichtsprozent eines Diamins auf Polyethylenexidbasis, 0,5 bis 1,5 Gewichtsprozent NaCl, 5-15 Gewichtsprozent Isocyanat, wobei diese Substanzen in einer wässrigen Pufferlösung, welche den pH-Wert im sauren Bereich puffern kann, enthalten sind. Vorzugsweise umfasst diese wässrige Pufferlösung dabei äquimolare Konzentrationen der Puffersubstanzen, also das gleiche Stoffmengenverhältnis von Säure und Base.

Um das Gel zur Wundbehandlung einzusetzen, wird eine Wundauflage verwendet, die das Hydrogel in einer Wundkontaktschicht bereithält. Auf diese Weise werden die den pH-Wert im sauren Bereich stabilisierenden Eigenschaften des Gels in der Wunde bereitgestellt.

Die im Rahmen des erfindungsgemäßen Herstellungsverfahrens vorgesehenen Puffersubstanzen weisen bevorzugt pKs-Werte zwischen 3,5 und 6,9 auf. Beispiele für solche Puffersubstanzen sind Essigsäure/Acetat (pKs 4,76), Benzoesäure/Benzoat (pKs 4,2) oder Milchsäure/Lactat (pKs 3,9). Die vorgenannten Puffersysteme haben insbesondere den Vorteil, dass sie in zahlreichen Produkten der Lebensmittelindustrie verwendet werden und daher lange erprobt sind. Außerdem sind sie auf einfache und kostengünstige Weise auf chemischem Wege zu synthetisieren.

Bei der Benutzung des erfindungsgemäßen Wundversorgungsprodukts sorgt insbesondere die Exsudation der Wunde für eine gute Lösung und Verteilung der Puffersubstanzen in der Wunde.

Nachstehend werden Ausführungsformen erfindungsgemäßer Wundversorgungsprodukte anhand von Zeichnungen näher erläutert. Die Erfindung soll jedoch nicht auf die in den Zeichnungen oder in der Beschreibung der Zeichnung dargestellten Ausgestaltungen reduziert verstanden werden. Es zeigen:
Figur 1: Querschnitt durch ein mehrschichtiges Wundversorgungsprodukt
Figur 2: Querschnitt durch eine weitere Ausführungsform eines mehrschichtigen Wundversorgungsproduktes
Figur 3: Draufsicht auf die im Gebrauch der Wunde zugewandte Seite des mehrschichtigen Wundversorgungsprodukts aus Figur 1

| **Bezugszeichen** | **Bedeutung** |
|---|---|
| **10** | **Wundversorgungsprodukt** |
| **11** | **Polyurethanschaum** |
| **12** | **Polyurethan-Deckschicht** |
| **13** | **Klebstoff** |
| **15** | **Hydrogel** |
| **16** | **Kleberand** |
| **20** | **Wundversorgungsprodukt mit Hydrogel** |

### Figur 1:

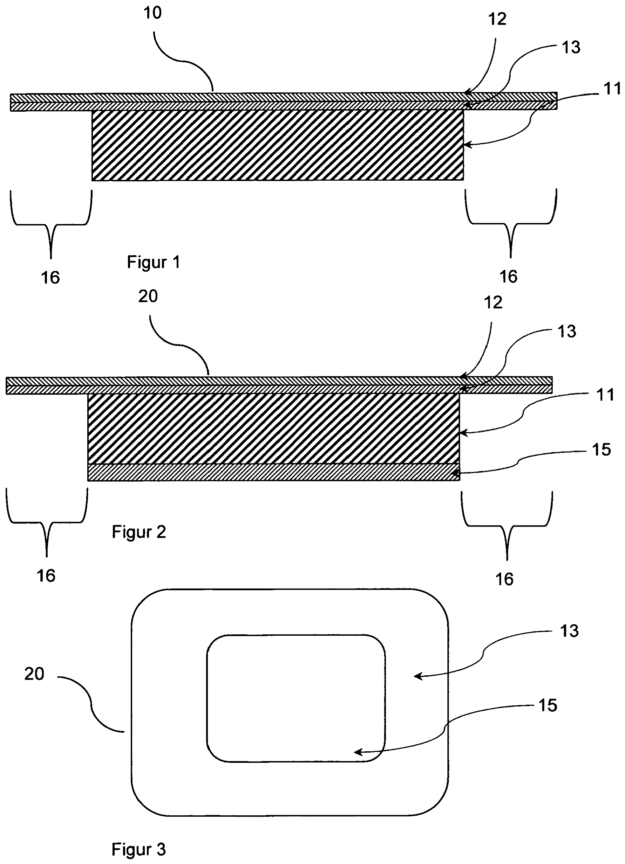

Das in Figur 1 schematisch dargestellte Wundversorgungsprodukt 10 (nicht erfindungsgemäß) umfasst eine mehrschichtige Wundauflage. Diese weist als Trägermaterial eine elastische, wasserdampf-durchlässigen Deckschicht 12 aus Polyurethan auf, die auf einer Seite mit einem Acrylatkleber 13 versehen ist. Die Polyurethan-Deckschicht 12 ragt über einen weichen, hydrophilen Polyurethan-Schaum 11 hinaus und bildet so einen Kleberand 16, mit dem das Produkt an der Haut des Patienten fixiert werden kann. Die Polyurethan-Deckschicht 12 weist vorzugsweise Abmessungen von 15 cm x 15 cm auf, wobei mittig auf der mit Acrylatkleber 13 versehenen Seite der Polyurethan- Deckschicht 12 der Polyurethan-Schaum 11 mit einer Größe von 10 x 10 cm vorgesehen ist.

Der Polyurethan-Schaum 11 weist eine Dichte von 70 bis 110 kg/m³ auf. Die Porengröße des Schaums weist einen Gradienten mit zunehmend kleineren Poren von der Wundseite hin zur wundabgewandten Seite der Schaumlage auf und beträgt durchschnittlich 100 bis 300 µm. Der Polyurethan-Schaum 11 wurde durch Imprägnierung mit den Puffersubstanzen Benzoesäure und Benzoat (Konzentration 0,04 M) versehen, um eine Stabilisierung des pH-Werts im pH-Bereich pH 4 bis pH 4,5 und eine Pufferkapazität von etwa 0,3 - 0,4 mol NaOH zu erreichen. Nach der Imprägnierung wurde eine vollständige Trocknung des Polyurethan-Schaums 11 vorgenommen, so dass das Wundversorgungsprodukt 10 vor der Benutzung trocken vorliegt.

Denkbar und vorteilhaft wäre es außerdem, feuchte Schäume mit Puffereigenschaften herzustellen, welche insbesondere zur Behandlung von trockenen Wunden geeignet sind. In einer Variante des Wundversorgungsprodukts 10 wird der Polyurethan-Schaum 11 nach dem Imprägnieren nicht vollständig getrocknet, sondern weist nach dem Trocknungsvorgang noch Pufferlösung in der Polymermatrix der Schaumschicht auf. In diesem Fall kann ein kürzerer Zeitraum für die Trocknung des Schaums nach dem Imprägnieren vorgesehen werden, wodurch der Schaum eine Restfeuchte aufweist. Die Restfeuchte des Polyurethan-Schaums 11 beträgt bevorzugt zwischen 4 g und 12 g Flüssigkeit pro g Schaum. Eine Restfeuchte von 4 g bis 12 g Flüssigkeit pro g Schaum entspricht in etwa der Menge an Flüssigkeit, die ein hydrophiler Polyurethan-Schaum 11 durch Aufquellen in seiner Polymermatrix zurückhalten kann. Ein Polyurethan-Schaum 11 mit einer Restfeuchte von 4 g bis 12 g Wasser pro g Schaum enthält wenig Flüssigkeit, so dass der Polyurethan-Schaum 11 weiterhin gute Absorptionsfähigkeiten behält. Ein Wundversorgungsprodukt 10, welches einen Polyurethan-Schaum 11 mit Restfeuchte aufweist, versorgt eine Wunde von Beginn der Behandlung an mit Feuchtigkeit. Außerdem weist das vorgenannte Wundversorgungsprodukt einen absorbierenden Polyurethan-Schaum 11 mit einer ausreichenden Absorptionskapazität auf. Die Absorption erfolgt, indem der Polyurethan-Schaum 11 Wundexsudat in seine Poren aufsaugt. Dabei hat es sich als besonders vorteilhaft erwiesen, wenn der Polyurethan-Schaum 11 einen Kapillareffekt erzeugen kann. Ein Kapillareffekt kann beispielsweise mittels eines Gradienten der Porengröße erzeugt werden. Ein solcher Gradient der Porengröße ergibt sich durch eine sich von einer Seite der Polyurethan-Schaumschicht 11 zur anderen Seite der Polyurethan-Schaumschicht 11 verringernde durchschnittliche Porengröße. Bevorzugt ist es, wenn die im Gebrauch der Wunde zugewandte Seite größere Poren als die der Wunde abgewandte Seite aufweist. Durch die Absorption von Wundexsudat werden Substanzen wie z.B. Abbauprodukte des Wundgewebes oder zytotoxische Ausscheidungen von Bakterien, die die Wundheilung möglicherweise negativ beeinflussen können, der Wundoberfläche entzogen. Gleichzeitig wird Feuchtigkeit in einer ausreichenden Menge zur Verfügung gestellt.

Polyurethan-Schäume 11 mit einer Restfeuchte weisen im Vergleich mit trockenen Schäumen eine geringere Absorptionskapazität auf. Trockene Polyurethan-Schäume 11, insbesondere wenn keine freisetzbaren Puffersubstanzen enthalten sind, können schnell große Mengen Wundexsudat aufnehmen. Die schnelle Absorption von Wundexsudat kann zu einer trockenen Wundoberfläche führen. Trockene Wundoberflächen behindern einen schnellen Wundheilungsverlauf und können die Narbenbildung begünstigen. Somit sind Wundversorgungsprodukte 10 mit feuchten Wundauflagen hervorragend geeignet, in der Epithelisierungs- oder Granulationsphase der Wundheilung eingesetzt zu werden. Damit kann die erfindungsgemäße Wundauflage 10 in natürlicher Weise die Granulation und/ oder die Epithelisierung der Wunde in besonderem Maße fördern.

### Figur 2:

Gemäß der in Figur 2 gezeigten Ausführungsform der Erfindung umfasst das Wundversorgungsprodukt 20 ein Hydrogel 15, welches zwischen Wunde und Polyurethan-Schaum 11 angeordnet ist. Das vorgenannte Hydrogel 15 weist eine Gesamtkonzentration von 0,05 M Zitronensäure und Zitrat als Puffersubstanzen auf. Das Hydrogel 15 ist geeignet, den pH-Wert einer Wunde im Bereich zwischen pH 4 bis pH 6 zu stabilisieren. Das Wundversorgungsprodukt 20 weist eine Pufferkapazität von 0,1-0,2 mol NaOH auf. Vorteilhaft ist es, wenn das Hydrogel 15 10-20 Gewichtsprozent Glycerin, 0,5-3 Gewichtsprozent Hydroxycellulose und 0,2-2 Gewichtsprozent Natriumchlorid umfasst. Die Polyurethan-Deckschicht 12 weist Abmessungen von 11 cm x 11 cm auf, wobei sich mittig auf der mit Acrylatkleber 13 versehenen Seite der Polyurethan-Deckschicht 12 eine Schaumschicht aus weichem, hydrophilem Polyurethan 11 mit einer Größe von 6 x 6 cm befindet.

Gemäß einer Ausführungsform des Wundversorgungsprodukts 20 wird ein Hydrogel 15 verwendet, welches 20-60 Gewichtsprozent Propylenglykol, 3-10 Gewichtsprozent eines Diamins auf Polyethylenexidbasis, 0,5 bis 1,5 Gewichtsprozent NaCl und 5-15 Gewichtsprozent Isocyanat umfasst.

In dem in Figur 2 gezeigten Wundversorgungsprodukts 20 enthalten der Polyurethan-Schaum 11 und das Hydrogel 15 Puffersubstanzen. Durch die Kombination aus einem Polyurethan-Schaum 11 und einem Hydrogel 15, wobei sowohl der Polyurethan-Schaum 11 und das Hydrogel 15 Puffersubstanzen für die Stabilisierung des pH-Werts einer Wunde im sauren pH-Bereich aufweisen, kann eine besonders hohe Pufferkapazität erreicht werden. Somit ist das hier beschriebene Ausführungsbeispiel besonders für stark exsudierende Wunden geeignet, deren pH-Wert durch Entzündungsprozesse und/oder eine bakterielle Infektionen in den alkalischen pH-Bereich steigen könnte.

### Figur 3:

Die Wundseite des Wundversorgungsprodukts 20 aus Figur 2 ist in der Draufsicht abgebildet.

Im Folgenden werden Versuche zu den pH-Wert stabilisierenden Eigenschaften erfindungsgemäßer Wundversorgungsprodukte erläutert.

Bei einem erfindungsgemäßen Wundversorgungsprodukt, welches Puffersubstanzen und eine Wundauflage mit einer Schaumschicht umfasst, kann die Abgabe von Puffersubstanzen in die Wunde durch ein einfaches Testsystem mittels in-vitro Titrationsexperimenten gemessen werden. Die Fähigkeit des erfindungsgemäßen Wundversorgungsprodukts, einen pH-Wert in einem sauren pH-Bereich zu stabilisieren, wird mittels der Pufferkapazität beschrieben. Je höher die Pufferkapazität ist, desto mehr Wundexsudat kann durch das erfindungsgemäße Wundversorgungsprodukt im sauren pH-Bereich stabilisiert werden.

Zur Messung der Pufferkapazität des in dem Wundversorgungsprodukt enthaltenen Schaums wurde 1 g der Schaumlage des erfindungsgemäßen Wundversorgungsprodukts in ein Wasserbad mit 15 ml demineralisiertem Wasser gelegt. Das Wasserbad mit dem Wundversorgungsprodukt wurde 24 Stunden lang bei Raumtemperatur (25°C) auf einem Schüttler mit 100 bpm (Schüttelbewegungen pro Minute) geschüttelt. Die Puffersubstanzen konnten sich während der 24 Stunden aus der Schaumschicht in das demineralisierte Wasser lösen und eine Flüssigkeit mit pH-Wert stabilisierenden Eigenschaften bilden. Anschließend wurde die Flüssigkeit, welche Puffersubstanzen enthielt, zusammen mit dem Produkt automatisiert titriert. Durch den Verbrauch an NaOH konnte die Pufferkapazität bestimmt werden. Es wurden jeweils Dreifachbestimmungen durchgeführt. Es konnte beobachtet werden, dass der pH-Wert stabilisierende Effekt über die Versuchsdauer von 24 Stunden weitgehend erhalten bleibt.
Die Pufferkapazität des in dem Wundversorgungsprodukt enthaltenen Hydrogelanteils, sofern vorhanden, wurde auf analoge Weise ermittelt, d.h. es wurde 1 g der Gellage in ein Wasserbad mit 15 ml demineralisiertem Wasser gelegt. Die Freisetzung der Puffersubsubstanzen aus der Gellage und die Bestimmung der Pufferkapazität erfolgte wie vorstehend für die Schaumlage beschrieben.

Als vorteilhaft für eine gewebeschonende Stabilisierung des pH-Werts im sauren pH-Bereich haben sich in Vorexperimenten Konzentrationen der Puffersubstanzen von 0,01 M bis 0,6 M erwiesen. Es zeigte sich, dass ein Polyurethanschaum (PermaFoam von Paul Hartman AG) eine besonders hohe Pufferkapazität von (0,72 +/- 0,05) mol NaOH aufwies (siehe Tabelle 1), wenn für die Imprägnierung die Puffersubstanzen Zitronensäure und Zitrat (Konzentration 0,05 M) verwendet wurden. Die Puffersubstanzen Benzoesäure und Benzoat (Konzentration 0,04 M) wiesen eine geringere Pufferkapazität von (0,33 +/- 0,02) mol NaOH auf. Die Puffersubstanzen Milchsäure / Lactat (Konzentration 0,05 M) wiesen gleichfalls eine geringere Pufferkapazität von 0,30 mol +/- 0,01 mol NaOH auf. Alle Beispiele in Tabelle 1 betreffen kein erfindungsgemäßes Wundversorgungsprodukt.

**Tabelle 1: Pufferkapazität von mit Puffersubstanzen imprägnierten Schaumlagen und mit Puffersubstanzen versetzten Gelen in Abhängigkeit von den verwendeten Puffersubstanzen und der Konzentration der Puffersubstanzen. σ: empirische Standardabweichung.**

| **puffernde Schicht** | **Puffersubstanzen** | **Konzentration der Puffersubstanzen [M]** | **Pufferkapazität [mol NaOH]** | **+/- σ [mol NaOH]** |
|---|---|---|---|---|
| Polyurethanschaum | Zitronensäure / Citrat | 0,05 | 0,72 | 0,05 |
| Polyurethanschaum | Zitronensäure / Citrat | 0,10 | 1,51 | 0,02 |
| Polyurethanschaum | Benzoesäure / Benzoat | 0,02 | 0,16 | 0,01 |
| Polyurethanschaum | Benzoesäure / Benzoat | 0,04 | 0,33 | 0,02 |
| Polyurethanschaum | Milchsäure / Lactat | 0,05 | 0,30 | 0,01 |
| Polyurethanschaum | Milchsäure / Lactat | 0,10 | 0,62 | 0,05 |
| Hydrogel | Zitronensäure / Citrat | 0,05 | 0,13 | 0,01 |
| Hydrogel | Milchsäure / Lactat | 0,10 | 0,11 | 0,01 |

In weiteren Messungen wurde bestimmt, wie schnell sich die Puffersubstanzen aus den Schaumlagen und Hydrogelen in demineralisiertem Wasser lösen können. Hierzu wurde eine 1 g Probe der Schaumlage bzw. des Hydrogels des erfindungsgemäßen Wundversorgungsprodukts in 15 ml demineralisiertes Wasser gegeben und das Wasserbad mit dem Wundversorgungsprodukt bei Raumtemperatur (25°C) auf einem Schüttler mit 100 bpm (Schüttelbewegungen pro Minute) geschüttelt. Für jede Messreihe wurden vier Proben in je einem Wasserbad geschüttelt. Die Messreihen wurden zuerst an Schaumlagen durchgeführt.

Bei der ersten Probe wurde die Pufferkapazität der Flüssigkeit, welche Puffersubstanzen enthielt, im ersten Wasserbad mittels Titration mit NaOH nach einer Stunde gemessen. Es zeigte sich, dass sich bei der ersten Probe, also bereits nach einer Stunde, mindestens 90% der nach 24 Stunden messbaren maximalen Pufferkapazität entfaltet hatte. Nach einer Stunde waren also mehr als 90% der aus dem Schaum in die Flüssigkeit lösbaren Puffersubstanzen in der Flüssigkeit gelöst.

Bei der zweiten Probe im zweiten Wasserbad wurde die Titration nach 3 Stunden durchgeführt. Die Pufferkapazität betrug über 90% der nach 24 Stunden messbaren maximalen Pufferkapazität.

Bei der dritten Probe im dritten Wasserbad wurde die Titration nach 6 Stunden durchgeführt. Die Pufferkapazität betrug gleichfalls über 90% der nach 24 Stunden messbaren maximalen Pufferkapazität.

Bei der vierten Probe im vierten Wasserbad wurde die Titration nach 24 Stunden durchgeführt. Die bei dieser Messung bestimmte Pufferkapazität wurde als 100% der nach 24 Stunden messbaren maximalen Pufferkapazität festgesetzt.

Im Rahmen der Messreihe an Schaumlagen zeigte sich, dass die Pufferkapazität bei den Messungen nach 1, 3 und 6 Stunden auf einem Plateau mit über 90% der nach 24 Stunden messbaren maximalen Pufferkapazität konstant blieb.

Bei den mit Puffersubstanzen versetzten Gelen wurden in analoger Weise Titrationsexperimente durchgeführt. Nach drei Stunden wurde eine vollständige Lösung der Puffersubstanzen aus dem Gel in der Flüssigkeit festgestellt, wobei die Pufferkapazität auch bei den dritten und vierten Proben, also nach 6 Stunden und 24 Stunden, konstant blieb.

## Patentansprüche

1. Wundversorgungsprodukt (20), umfassend Puffersubstanzen und eine Wundauflage, wobei die Wundauflage einen Schaum aufweist und
der pH-Wert der sich bei Lösung der Puffersubstanzen in demineralisiertem Wasser bei 37°C ergebenden Pufferlösung zwischen pH 3 und pH 7 liegt,
**dadurch gekennzeichnet, dass**
das Wundversorgungsprodukt (20) eine Wundkontaktschicht aufweist, welche ein Hydrogel umfasst, , wobei die Puffersubstanzen in dem Schaum und in dem Hydrogel vorgesehen sind, wobei die Puffersubstanzen Zitronensäure und Zitrat als Puffersubstanzen umfassen und wobei der Schaum ein hydrophiler Polyurethanschaum ist.

2. Wundversorgungsprodukt (20) nach Anspruch 1, wobei der pH-Wert der sich bei Lösung der Puffersubstanzen in demineralisiertem Wasser bei 37°C ergebenden Pufferlösung zwischen pH 3,5 und pH 6 liegt.

3. Wundversorgungsprodukt (20) nach Anspruch 1, wobei der pH-Wert der sich bei Lösung der Puffersubstanzen in demineralisiertem Wasser bei 37°C ergebenden Pufferlösung zwischen pH 3,8 und pH 5 liegt.

4. Wundversorgungsprodukt (20) nach einem der vorhergehenden Ansprüche, wobei die Puffersubstanzen in dem Schaum homogen verteilt vorliegen.

5. Wundversorgungsprodukt (20) einem der vorhergehenden Ansprüche, wobei die Pufferkapazität des Schaums bei einer Konzentration der Puffersubstanzen von 0,05 M mindestens 0,25 mol NaOH, insbesondere mindestens 0,55 mol NaOH beträgt.

6. Wundversorgungsprodukt (20) nach Anspruch 4, wobei die Pufferkapazität des Hydrogels bei einer Konzentration der Puffersubstanzen von 0,05 M mindestens 0,08 mol NaOH, insbesondere mindestens 0,1 mol NaOH beträgt.

7. Wundversorgungsprodukt (20) nach einem der vorhergehenden Ansprüche, wobei die Pufferkapazität des Wundversorgungsprodukts (20) nach spätestens drei Stunden 90% der nach 24 Stunden messbaren maximalen Pufferkapazität erreicht.

8. Verfahren zu Herstellung eines Wundversorgungsprodukts (20), umfassend die Schritte:
a. Zur Verfügung stellen eines Schaums
b. Zur Verfügung stellen einer Pufferlösung mit Puffersubstanzen, wobei der pH-Wert der Pufferlösung bei 37°C zwischen pH 3 und pH 7 liegt
c. Imprägnieren des Schaums mit der Pufferlösung
d. Vollständiges oder teilweises Trocknen des Schaums
e. Zur Verfügung stellen weiterer Schichten
f. Zusammenfügen des Schaums und der weiteren Schichten zu einer mehrschichtigen Wundauflage,
sowie umfassend die Schritte:
a. Zur Verfügung stellen eines Gels
b. Zur Verfügung stellen einer Wundauflage, wobei die Wundauflage einen Schaum aufweist
c. Zur Verfügung stellen einer Pufferlösung mit Puffersubstanzen, wobei der pH-Wert der Pufferlösung bei 37°C zwischen pH 3 und pH 7 liegt.
d. Vermischen des Gels mit der Pufferlösung
e. Zusammenfügen des Gels und der Wundauflage zu einer mehrschichtigen Wundauflage.

## Claims

1. Wound care product (20) comprising buffer substances and a wound dressing, the
wound dressing comprising a foam and
the pH of the buffer solution resulting upon dissolution of the buffer substances in demineralized water at 37°C being between pH 3 and pH 7,
**characterized in that**
the wound care product (20) comprises a wound contact layer which comprises a hydrogel, wherein the buffer substances are provided in the foam and in the hydrogel, wherein the buffer substances comprise citric acid and citrate as buffer substances and wherein the foam is hydrophilic polyurethane foam.

2. Wound care product (20) according to Claim 1, wherein the pH of the buffer solution resulting upon dissolution of the buffer substances in demineralized water at 37°C is between pH 3.5 and pH 6.

3. Wound care product (20) according to Claim 1, wherein the pH of the buffer solution resulting upon dissolution of the buffer substances in demineralized water at 37°C is between pH 3.8 and pH 5.

4. Wound care product (20) according to any of the preceding claims, wherein the buffer substances are distributed homogeneously in the foam.

5. Wound care product (20) according to any of the preceding claims, wherein the buffering capacity of the foam is, at a concentration of the buffer substances of 0.05 M, at least 0.25 mol of NaOH, more particularly at least 0.55 mol of NaOH.

6. Wound care product (20) according to Claim 4, wherein the buffering capacity of the hydrogel is, at a concentration of the buffer substances of 0.05 M, at least 0.08 mol of NaOH, more particularly at least 0.1 mol of NaOH.

7. Wound care product (20) according to any of the preceding claims, wherein the buffering capacity of the wound care product (20) reaches 90% of the maximum buffering capacity measurable after 24 hours after no later than three hours.

8. Method for producing a wound care product (20), comprising the steps of:
a. providing a foam
b. providing a buffer solution containing buffer substances, the pH of the buffer solution at 37°C being between pH 3 and pH 7
c. impregnating the foam with the buffer solution
d. completely or partially drying the foam
e. providing further layers
f. joining the foam and the further layers to form a multilayered wound dressing,
as well as comprising the steps of:
a. providing a gel
b. providing a wound dressing comprising a foam
c. providing a buffer solution containing buffer substances, the pH of the buffer solution at 37°C being between pH 3 and pH 7.
d. mixing the gel with the buffer solution
e. joining the gel and the wound dressing to form a multilayered wound dressing.

## Revendications

1. Produit de soin de plaie (20), comprenant des substances tampon et un pansement,
le pansement présentant une mousse et
la valeur du pH de la solution tampon obtenue lors de la dissolution des substances tampon dans de l'eau déminéralisée à 37°C se situant entre pH 3 et pH 7,
**caractérisé en ce que**
le produit de soin de plaie (20) présente une couche de contact avec la plaie, qui comprend un hydrogel, les substances tampon se trouvant dans la mousse et dans l'hydrogel, les substances tampon comprenant de l'acide citrique et du citrate en tant que substances tampon et la mousse étant une mousse de polyuréthanne hydrophile.

2. Produit de soin de plaie (20) selon la revendication 1, la valeur du pH de la solution tampon obtenue lors de la dissolution des substances tampon dans de l'eau déminéralisée à 37°C se situant entre pH 3,5 et pH 6.

3. Produit de soin de plaie (20) selon la revendication 1, la valeur du pH de la solution tampon obtenue lors de la dissolution des substances tampon dans de l'eau déminéralisée à 37°C se situant entre pH 3,8 et pH 5.

4. Produit de soin de plaie (20) selon l'une quelconque des revendications précédentes, les substances tampon se trouvant dans la mousse selon une répartition homogène.

5. Produit de soin de plaie (20) selon l'une quelconque des revendications précédentes, la capacité tampon de la mousse à une concentration en substances tampon de 0,05 M étant d'au moins 0,25 mole de NaOH, en particulier d'au moins 0,55 mole de NaOH.

6. Produit de soin de plaie (20) selon la revendication 4, la capacité tampon de l'hydrogel à une concentration en substances tampon de 0,05 M étant d'au moins 0,08 mole de NaOH, en particulier d'au moins 0,1 mole de NaOH.

7. Produit de soin de plaie (20) selon l'une quelconque des revendications précédentes, la capacité tampon du produit de soin de plaie (20) atteignant après au plus tard trois heures 90% de la capacité tampon maximale mesurable après 24 heures.

8. Procédé pour la fabrication d'un produit de soin de plaie (20) comprenant les étapes de :
a. mise à disposition d'une mousse,
b. mise à disposition d'une solution tampon présentant des substances tampon, la valeur du pH de la solution tampon à 37°C se situant entre pH 3 et pH 7,
c. imprégnation de la mousse par la solution tampon,
d. séchage complet ou partiel de la mousse,
e. mise à disposition d'autres couches,
f. assemblage de la mousse et des autres couches en un pansement multicouche,
ainsi que comprenant les étapes de :
a. mise à disposition d'un gel,
b. mise à disposition d'un pansement, le pansement présentant une mousse,
c. mise à disposition d'une solution tampon présentant des substances tampon, la valeur du pH de la solution tampon à 37°C se situant entre pH 3 et pH 7,
d. mélange du gel avec la solution tampon,
e. assemblage du gel et des autres couches en un pansement multicouche.
